# EUROPÄISCHE PATENTSCHRIFT

(11) **EP 2 480 149 B1**
(45) Veröffentlichungstag und Bekanntmachung des Hinweises auf die Patenterteilung: **03.05.2017**
(21) Anmeldenummer: 10798988.1
(22) Anmeldetag: 06.12.2010
(51) Int. Cl.: A61B 17/70, A61B 17/80

(54) **STABVERBINDER ZUR QUERVERBINDUNG ZWEIER WIRBELSÄULENSTÄBE**
ROD CONNECTOR FOR CROSS-CONNECTING OF TWO SPINAL RODS
CONNECTEUR DE TIGES DE LIAISON TRANSVERSALE DE DEUX TIGES VERTÉBRALES

(30) Priorität: 10.12.2009 DE 102009056890
(43) Veröffentlichungstag der Anmeldung: 01.08.2012
(73) Patentinhaber: Kraus, Kilian, 97440 Werneck (DE)
(72) Erfinder: Kraus, Kilian, 97440 Werneck (DE)
(74) Vertreter: Meissner Bolte Partnerschaft mbB
(86) Internationale Anmeldenummer: PCT/EP2010/068969
(87) Internationale Veröffentlichungsnummer: WO 2011/069963

(56) Entgegenhaltungen:
- WO-A2-2007/133938
- WO-A2-2008/140756
- JP-A- 7 241 299
- US-A1- 2006 271 045
- US-A1- 2007 016 197
- US-A1- 2008 109 039

## Beschreibung

Die Erfindung betrifft einen Stabverbinder zur Querverbindung zweier Wirbelsäulenstäbe.

Mit Hilfe orthopädischer Operationen an Patienten werden unter anderem Korrekturen am Verlauf der Wirbelsäule durchgeführt. Hierzu werden während einer Operation in der Regel zwei Wirbelsäulenstäbe links und rechts der Wirbelsäule mit Hilfe verschiedener Haken oder Schrauben an der Wirbelsäule bzw. den Wirbeln befestigt. Um beide Wirbelsäulenstäbe auch sicher in Relation zueinander zu fixieren, sind sogenannte Stabverbinder (auch Stab-zu-Stab-Verbinder genannt) bekannt.

Aus der US 7,029,474 B2 ist ein Stabverbinder bekannt, welcher an den Stäben derart fixiert wird, dass ein Klemmbacken mit Hilfe einer Schraube gegen den Stab gedrückt wird. Aus der US 7,066,938B2 ist ein alternativer Stabverbinder bekannt, welcher nach Art einer expandierbaren Klammer auf den Stab aufgeschnappt wird. Die WO 2008/140756 betrifft einen Stabverbinder gemäß dem Oberbegriff des Anspruchs 1.

Aufgabe der vorliegenden Erfindung ist es, einen verbesserten Stabverbinder anzugeben.

Die Aufgabe wird gelöst durch einen Stabverbinder zur Querverbindung zweier Wirbelsäulenstäbe gemäß Patentanspruch 1. Der Stabverbinder umfasst zwei Klemmen, welche in einem Montagezustand je einen Wirbelsäulenstab befestigend teilweise umgreifen. Der Montagezustand bezeichnet denjenigen Zustand, wenn der Stabverbinder auf den Wirbelsäulenstäben aufgebracht ist; die erste Klemme umfasst dann den ersten Wirbelsäulenstab, die zweite Klemme den zweiten Wirbelsäulenstab. Die Klemmen umgreifen hierbei die Wirbelsäulenstäbe nur zum Teil, d.h. nicht um den vollen Umfang, jedoch soweit, dass die Klemmen sicher auf dem Wirbelsäulenstab befestigt sind.

Der Stabverbinder enthält außerdem einen die Klemmen relativ zueinander ortsfixierenden Quersteg. Der Quersteg kann hierbei beispielsweise starr ausgeführt sein, d.h. die Klemmen haben bezüglich der zu umgreifenden Wirbelsäulenstäbe eine festgelegte Orientierung zueinander, beispielsweise sind die Klemmen derart zueinander orientiert, dass umgriffene Wirbelsäulenstäbe im Montagezustand immer parallel verlaufen. In der Regel wird jedoch der Quersteg bezüglich der Relativposition der Klemmen verstellbar und in einer beliebigen bzw. gewünschten Relativposition arretierbar sein. Durch ein- und denselben Stabverbinder können so Wirbelsäulenstäbe in verschiedenen relativen Ortslagen zueinander fixiert werden.

Die Beschränkung auf zwei Klemmen ist nicht unbedingt zwingend notwendig. Denkbar wären auch Stabverbinder mit drei oder mehr Klemmen, welche dann jeweils durch den Quersteg relativ zueinander ortsfixiert sind und entweder ein Wirbelsäulenstab durch das Aufschnappen von zwei Klemmen besonders sicher arretiert werden soll oder auch ein dritter Wirbelsäulenstab durch einen einzigen Stabverbinder relativ zu zwei weiteren Stäben fixiert wird.

Der Stabverbinder enthält weiterhin eine Federeinrichtung für die Klemme. Mit anderen Worten ist die Klemme also federnd ausgebildet, um auf den Wirbelsäulenstab aufgeschnappt oder auch wieder von diesem entnommen zu werden. Die Klemme muss z.B. beim Aufsetzen auf den Wirbelsäulenstab bezüglich ihrer Backen auseinanderfedern und bei vollständig aufgesetzten Zustand wieder - den Wirbelsäulenstab umgreifend - zufedern. Hierbei liegt die Klemme insbesondere mit der Federkraft reibschlüssig am Wirbelsäulenstab an. Erfindungsgemäß ist die Federkraft derart gering ausgelegt, dass die Klemme händisch, z.B. von einem Operateur, auf den Wirbelsäulenstab aufschnappbar, im Montagezustand auf diesem händisch verschiebbar und auch wieder händisch vom Wirbelsäulenstab lösbar ist. Die Verschiebbarkeit bedeutet hier sowohl eine axiale als auch eine Verschiebbarkeit in Umfangsrichtung des Wirbelsäulenstabes, welcher in der Regel zylinderförmig ausgeführt ist.

Gemäß der Erfindung weist der Stabverbinder zusätzlich auch eine Fixiereinrichtung für die Klemme auf. Diese ist von der Federeinrichtung unabhängig betätigbar. Wird die Fixiereinrichtung betätigt, erzeugt sie eine zusätzliche Haltkraft, welche die Klemme auf den Wirbelsäulenstab ausübt, die zur Federkraft hinzutritt bzw. diese ersetzt. Die Haltekraft ist dabei derart groß, dass die Klemme im Montagezustand auf dem Wirbelsäulenstab fest fixiert ist. Eine feste Fixierung bedeutet in diesem Zusammenhang, dass keine Verschiebung oder Rotation der Klemme auf dem Wirbelsäulenstab mehr möglich ist; weder händisch noch später durch die Benutzung des Wirbelsaulenstabes im Patienten.

Mit anderen Worten wird erfindungsgemäß ein Stabverbinder mit einer Doppelfunktion vorgesehen. Die erste ist eine Justierfunkticn: Dank der Federeinrichtung ist der Stabverbinder derart ausgebildet, dass er während einer Operation zwar unverlierbar auf die Wirbelsäulenstäbe aufgeschnappt ist, durch Verschiebung, Bewegung, eventuelles Wiederabschnappen und Aufsetzen an einer anderen Position jedoch eine Justierung ermöglicht. Dennoch ist der Stabverbinder bereits mit einer gewissen Kraft auf den Wirbelsäulenstäben gehalten, so dass eine sichere, einfache und zuverlässige Justage in eine gewisse Endposition möglich ist.

Die zweite ist eine Fixierfunktion: Erst wenn die gewünschte Endposition gefunden ist, wird die Fixiereinrichtung betätigt und der Wirbelsäulenstab endgültig in der betreffenden Position fixiert. Im Falle eines verstellbaren Quersteges betrifft die Endfixierung auch die Endfixierung des Quersteges, d.h. die Endfixierung der Relativposition der Klemmen zueinander.

Der Quersteg enthält zwei Schenkel, wobei jeder der Schenkel an je einer der Klemmen angebracht ist. Der Quersteg enthält außerdem ein von beiden Schenkeln durchsetztes Verbindungsteil mit einer auf beide Schenkel einwirkenden Arretiervorrichtung. Im Falle von sich in deren Axialrichtung überlappenden Schenkeln kann hier ein Freiheitsgrad für die Platzierung des Verbindungsteils erreicht werden. Dieses ist im Überlappungsbereich der beiden Schenkel verschiebbar. Das verbindungsteil kann so an eine geeignete Stelle bezüglich des Patienten verschoben werden.

Die Arretiervorrichtung ist eine solche, welche einen arretierenden Reibschluss zwischen Schenkel und Verbindungsteil erzeugt. Mit anderen Worten werden hier die beiden Schenkel im Verbindungsteil mit einer derartig hohen Klemmkraft verklemmt, dass diese eine dauerhafte Relativfixierung der beiden Schenkel und damit der Klemmen bewirkt.

Das Verbindungsteil enthält einen Rahmen zur Aufnahme des ersten Schenkels und eine im Rahmen rotierbar gelagerte Kugel, wobei die Kugel vom zweiten Schenkel durchsetzt ist. Der erste Schenkel ist hierbei im Rahmen z.B. fest gelagert oder axial entlang der Schenkellängsachse verschiebbar. Durch die Lagerung des zweiten Schenkels in der Kugel und deren Rotierbarkeit ist der zweite Schenkel gegenüber dem ersten - im Rahmen der Rotationsmöglichkeiten der Kugel - verkipp-, verdreh- und rotierbar. In der Regel ist hierbei noch eine axiale Verschiebbarkeit des zweiten Schenkels in der Kugel möglich. Die Arretiervorrichtung arretiert dann auch die Kugel im Rahmen und die jeweilige Position der Schenkel im Rahmen und in der Kugel. Durch die Kugel kann damit der zweite Schenkel bezüglich des Ersten und des Rahmens um den Kugelmittelpunkt rotieren.

Die Kugel weist zur Arretierung des in ihr verlaufenden zweiten Schenkels einen komprimierbaren Spalt auf. Mit anderen Worten wird durch die Arretiervorrichtung die Kugel am Spalt komprimiert, so dass sich diese reibschlüssig fest um den sie durchsetzenden zweiten Schenkel schließt und diesen arretiert. Der Mit anderen Worten sitzt der Schenkel zwischen zwei durch den Spalt getrennten Kugelteilen, die aufeinander zu bewegbar sind, um den Schenkel verklemmend zu arretieren.

Die Arretiervorrichtung enthält ein sich am Rahmen abstützendes und den ersten Schenkel gegen die Kugel pressendes Klemmelement. So werden durch ein einziges Klemmelement sämtliche Teile der Arretiervorrichtung bzw. des Querstegs zueinander fixiert, da zum Einen das Klemmelement den ersten Schenkel gegen die Kugel presst und diese zueinander fixiert und gleichzeitig in der Kugel durch deren Kompression in dieser der erste Schenkel verklemmt und dadurch gehalten wird. Außerdem wird die Kugel dann schlussendlich gegen den Rahmen gepresst und somit sämtlich Teile zueinander fixiert. Mit anderen Worten werden also durch das Klemmelement beide Schenkel, die Kugel und der Rahmen im Verbindungsteil fixierend aneinandergepresst.

In einer bevorzugten Ausführungsform weist die Klemme einen Grundträger mit einem feststehenden Klemmbacken und weiterhin einen am Grundträger federnd gelagerten Federbacken auf. Die federnde Lagerung wird durch die Federeinrichtung bewirkt, welche den Federbacken federn gegenüber dem Grundträger bzw. Klemmbacken lagert. Die Fixiereinrichtung arretiert dann den Federbacken gegen den Klemmbacken bei ihrer Betätigung, so dass der Federbacken gegen den Grundträger bzw. Klemmbacken aus einer den Wirbelsäulenstab fixierenden Endstellung nicht mehr wegfedern kann.

In einer Variante dieser Ausführungsform ist die Federeinrichtung ein den Federbacken mit dem Grundträger verbindender Federsteg. Der Federsteg besitzt somit eine Doppelfunktion, nämlich zum Einen den Federbacken mit dem Grundträger zu verbinden um zum Anderen die oben beschriebene Federwirkung der Klemme zu erzeugen. Ein separates Federelement zur Federbelastung eines z.B. gelenkig gelagerten Federbackens am Grundträger ist dann nicht mehr notwendig.

Außerdem wird so folgendes möglich: In einer weiteren Variante dieser Ausführungsform sind Federbacken, Federsteg und Grundträger einstückig ausgebildet. Abgestimmt auf das Material der Klemme ist der Federsteg, welcher einen massiven Federbacken mit einem massiven Grundträger verbindet, beispielsweise hinreichend dünn ausgeführt. Z.B. ist der Federsteg ein aus einem Metall nach Art einer Blattfeder freigeschnittener verbindungssteg zwischen Federbacken und Grundträger.

In einer weiteren Variante dieser Ausführungsform ist der Federbacken und/oder der Federsteg aus dem Grundträger freigeschnitten. Durch die Dimensionierung des Freischnitts kann die Federeigenschaft des Federteils einfach bestimmt werden. Außerdem sind so Verbindungstechniken für vormals einzelne Teile überflüssig, um diese wieder einstückig zu verbinden, z.B. zu verschweißen.

In einer weiteren Ausführungsform der Erfindung weisen Grundträger und Federbacken je ein Formschlusselement auf. Bezüglich der Federeinrichtung weisen beide Formschlusselemente Spiel zueinander auf. Durch die Fixiereinrichtung sind die Formschlusselemente jedoch formschlüssig ineinander bewegbar. Mit anderen Worten sind die Formschlusselemente im Hinblick auf die Federeigenschaften der Klemme, d.h. durch alleiniges Auffedern der Klemme nicht miteinander in Eingriff zu bringen. Solange die Klemme nicht endfixiert ist, greifen die Formschlusselemente nicht ineinander. Eine freie Federbewegung der Klemme ist möglich. Wird die Klemme endfixiert, greifen die Formschlusselemente ineinander und sorgen für einen sicheren und dauerhaften Halt der Klemme am Wirbelsäulenstab.

In einer Variante dieser Ausführungsform weisen die Formschlusselemente an ihren aneinander zugewandten Seiten eine zumindest annähernde S-förmige Passform auf. Mit anderen Worten ist ein zwischen Grundträger und Federbacken im Bereich der Formschlusselemente vorhandener Spalt S-förmig. Durch Verformung bzw. Bewegung des Federbackens mit Hilfe der Fixiereinrichtung werden beide Bauteile so aufeinander zubewegt, dass sich die Formschlusselemente berühren. Die S-förmige Passform bietet hierbei die Möglichkeiten eines formschlüssigen Verhakens der jeweiligen beiden U- bzw. bogenförmigen Teile der S-Form.

In einer bevorzugten Ausführungsform der Erfindung enthält die Fixiereinrichtung ein das Aufschwenken der Klemme verhinderndes Formschlusselement. Durch ein entsprechendes Formschlusselement wird tatsächlich eine Bewegung der Klemme hinsichtlich ihres Aufschwenkens formschlüssig verhindert, so dass diese auf dem Wirbelsäulenstab endfixiert verbleibt. Mit anderen Worten verhindert dann ein Formschluss das ansonsten durch das Federelement gegebene Spiel der Klemme. Als Beispiel für ein Formschlusselement wäre in Verbindung mit der oben genannten Ausgestaltung eines Grundträgers mit einem Federbacken ein Formschlusselement denkbar, welches den Federbacken gegenüber dem Grundträger formschlüssig abstützt. Das Formschlusselement ist in bevorzugter Weise unverlierbar durch eine Verliersicherung an der Klemme gehalten.

In einer Variante dieser Ausführungsform ist das Formschlusselement ein auf die Klemme einwirkendes Gewindelement. Mit anderen Worten wird durch Betätigung des Gewindelements ein Formschluss und insbesondere eine hohe Anpresskraft der Klemme am Wirbelsäulenstab erzeugt. Z.B. wird das Gewindeelement aus einer Position, in der es einen Abstand zum Federbacken aufweist, und so dessen Federbewegung ermöglicht, durch Einschrauben an diesen angelegt bzw. angepresst, so dass die vormalige Federbewegung nicht mehr möglich ist. Ein Gewindeelement ist z.B. eine Schraube bzw. ein im Grundträger gelagerter Gewindestift, der sich am Grundträger abstützt und gegen den Federbacken drückt. Z.B. ist dann gemäß oben die Schraube durch eine Verliersicherung am Grundträger gehalten, so dass diese sich auch im die Klemme nicht arretierenden Zustand sich nicht vom Stabverbinder lösen kann.

Für eine weitere Beschreibung der Erfindung wird auf die Ausführungsbeispiele der Zeichnungen verwiesen: Es zeigen, jeweils in einer schematischen Prinzipskizze:
- Fig. 1: einen auf zwei Wirbelsäulenstäben angebrachten Stabverbinder in perspektivischer Darstellung,
- Fig. 2: den längsmittig aufgeschnittenen Stabverbinder aus Fig.1 in perspektivischer Darstellung,
- Fig. 3: den entlang der Linie III-III quergeschnittenen Schenkel der unmontierten Klammer aus Fig. 1. in perspektivischer Darstellung,
- Fig. 4: den unmontierten Rahmen aus Fig. 1 in perspektivischer Darstellung,
- Fig. 5: die Vorderansicht des Rahmens aus Fig. 4 in Richtung des Pfeils V,
- Fig. 6: die Draufsicht des Rahmens aus Fig.4 in Richtung des Pfeils VI,
- Fig. 7: die unmontierte Kugel aus Fig. 1 in perspektivischer Darstellung,
- Fig. 8: einen Schnitt durch die Kugel aus Fig. 7 in Richtung des Pfeils VIII-VIII in Fig. 9,
- Fig. 9: die Vorderansicht der Kugel aus Fig. 7 in Richtung des Pfeils IX,
- Fig.10: Kugel und Rahmen in Montagestellung in perspektivischer Darstellung,
- Fig.11: die in den Rahmen eingeschobene Kugel in Richtung des Pfeils XI in Fig.10,
- Fig.12: die Situation aus Fig. 11 in Blickrichtung des Pfeils XII,
- Fig.13: die gegenüber der Situation aus Fig. 12 in die Innenfläche abgesenkte Kugel in einer Darstellung gemäß Fig. 12,
- Fig.14: die gegenüber der Situation aus Fig. 13 rotierte Kugel in einer Darstellung gemäß Fig. 12.

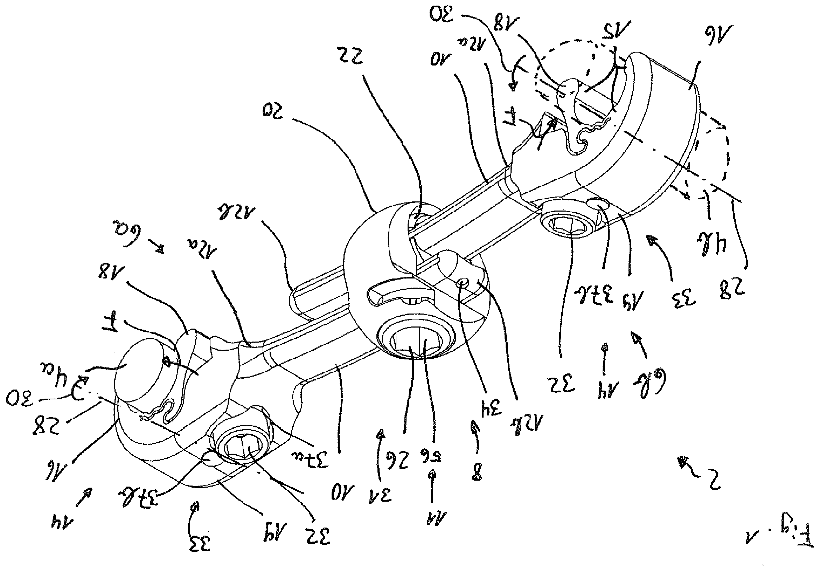

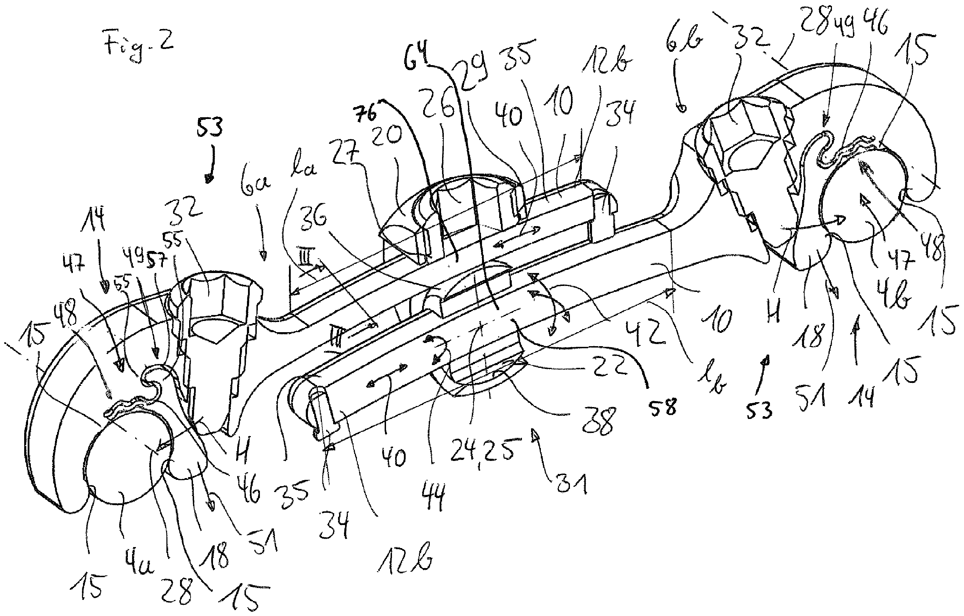

Fig. 1 und Fig. 2 zeigen einen Stabverbinder 2, der auf zwei Wirbelsäulenstäben 4a,b montiert ist. Der Stabverbinder ist im wesentlichen dreiteilig aufgebaut und umfasst zwei Klammern 6a,b und ein Verbindungsteil 8. Durch letzteres sind die Klammern 6a,b bezüglich ihrer Raumlage zueinander in mehreren Freiheitsgraden variierbar und in einer gewünschten Raumlage zueinander fixierbar. Jede der Klammern 6a,b weist einen sich länglich erstreckenden Schenkel 10 auf, an dessen jeweiligem einen Ende 12a eine Klemme 14 angebracht ist. Das jeweilige andere Ende 12b der Schenkel 10 liegt jenseits des Verbindungsteils 8, d.h. die Schenkel 10 durchsetzen dieses. Mit anderen Worten sind die Schenkel 10 einander zugewandt und überlappen sich. Die Schenkel 10 zusammen mit dem Verbindungsteil 8 bilden damit einen Quersteg 11, der die beiden Klemmen zueinander ortsfixiert.

Jede Klemme 14 ist aufgebaut aus einem Klemmbacken 16 und einem einstückig an diesen angeformten, federnd mit dem Klemmbacken 16 verbundenen Federbacken 18. Der Klemmbacken 16 ist mit anderen Worten Teil eines Grundträgers 19, an dem der Federbacken 18 gelagert ist und der Schenkel 10 fest anschließt.

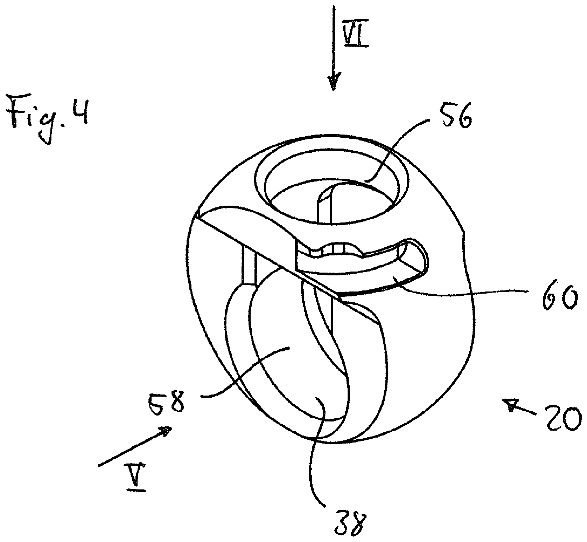

Das Verbindungsteil 8 umfasst einen Rahmen 20 und eine in diesem nach Art eines Kugelgelenks gelagerte Kugel 22. Die Kugel 22 weist eine einen Teil einer Kugel bildende Außenfläche 36, deren Mittelpunkt 24 der Kugelmittelpunkt ist. Der Rahmen 20 weist in seinem Inneren bzw. in einer Einschuböffnung 58 (s. auch Fig.4) eine die Außenfläche 36 nach Art eines Gleitlagers aufnehmende Innenfläche 38 auf, die ebenfalls Teil einer Kugelfläche mit gleichem Radius ist. Der Mittelpunkt 25 der Innenfläche 38 ist wieder deren entsprechender Kugelmittelpunkt. Da, wie etwa aus Fig.2 ersichtlich ist, die Kugel 22 in der Innenfläche 38 einliegt, fallen in dieser Situation die Kugelmittelpunkte 24,25 konzentrisch zusammen. Außenfläche 36 und Innenfläche 38 wirken daher im Sinne einer Gleitpaarung zusammen und bilden ein prinzipiell in alle Richtungen rotierbares Kugelgelenk.

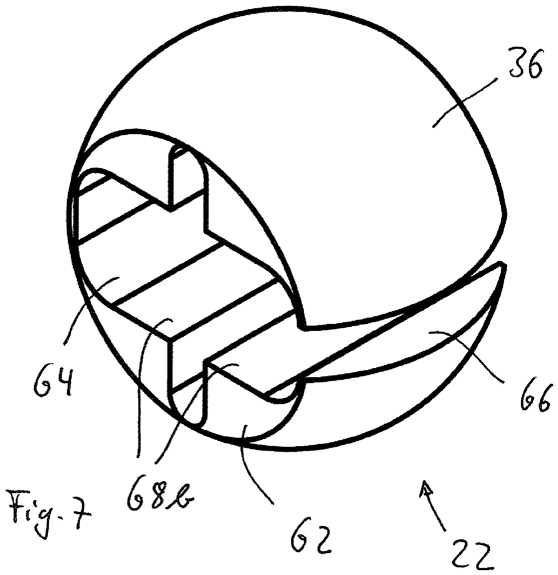

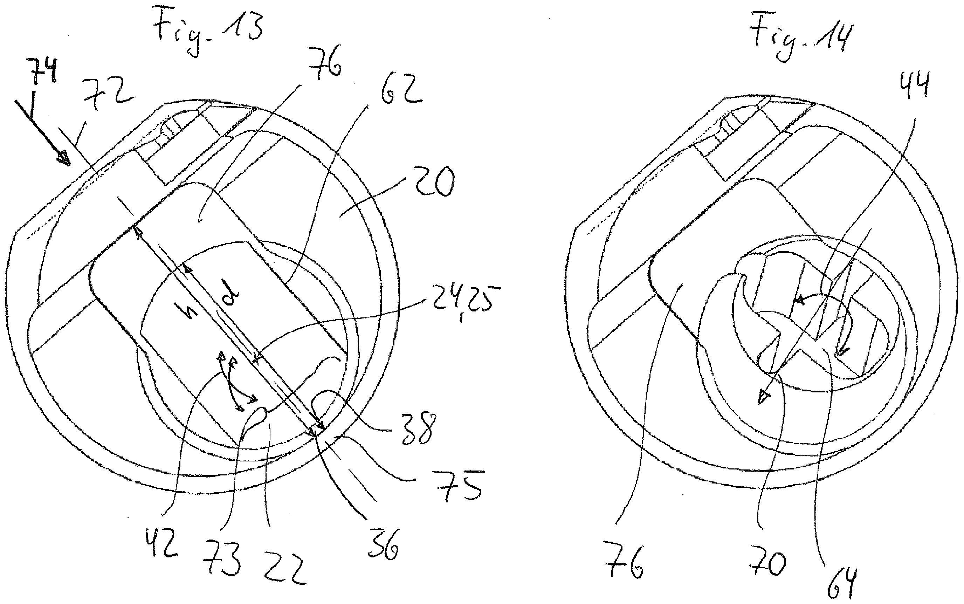

Die Kugel 22 weist einen Aufnahmeraum 64 (s. Fig.7) auf, der vom Schenkel 10 der Klammer 6b durchsetzt ist. Im Aufnahmeraum 64 ist der Schenkel 10 nach Art einer Gleitführung alleine in Richtung des Pfeils 40 axial verschiebbar geführt. Der Schenkel 10 der Klammer 6a befindet sich zwischen Kugel 22 und Rahmen 20 in einer weiteren Einschuböffnung bzw. in einem zwischen Kugel 22 und Rahmen 20 gebildeten Aufnahmeraum 76 (s. Fig.13). Die Schenkel 10 und die Kugel 22 sind durch Anziehen eines Klemmelements 26 in Form eines Gewindestiftes, welcher Teil des Verbindungsteils 8 ist, gegeneinander in ihrer räumlichen Lage fixierbar. Hierzu wird in der Regel ein Werkzeug benutzt. Das Klemmelement 26 bildet damit eine Arretiereinrichtung 31 zur Fixierung der Schenkel 10 und des Verbindungsteils 8 in einer gewünschten relativen Lage zueinander.
Das Klemmelement 26 weist an seinem inneren Ende einen Bund 27 auf, d.h. das Außengewinde des Klemmelements 26 ist nicht bis zum inneren Ende hin ausgebildet bzw. abgedreht. In der gezeigten Position in Fig. 2 stößt der Bund 27 an das innere Ende 29 des in einer Gewindeöffnung 56 eingebrachten Innengewindes an. Das Klemmelement 26 kann daher beim Zusammenbau des Verbindungsteils 8 nur von der Innenseite, also der Einschuböffnung 58 her, in die Gewindeöffnung 56 eingeschraubt werden. Nach dem vollständigen Zusammenbau des Verbindungsteils 8 ist das Klemmelement 26 damit unverlierbar gehalten. So kann das Klemmelement 26 weder bei der Handhabung des Stabverbinders 2 während einer Operation noch im in den Patienten eingesetzten Zustand verloren gehen, sollte sich dieser lockern.
Der Kraftschluss zur Fixierung verläuft im Detail folgendermaßen: Das in den Rahmen 20 eingreifende Klemmelement 26 presst den Schenkel 10 der Klammer 6a gegen die Außenfläche 36 der Kugel 22 und komprimiert diese aufgrund eines in ihr vorgesehenen Spaltes 66 (siehe Fig.7), so dass diese gegen den einliegenden Schenkel 10 der Klammer 6b drückt und dieser wiederum die gegenüberliegende Seite der Kugel 22 gegen die Innenfläche 38 des Rahmens 20 verklemmt.
Da die Kugel 22 um den gemeinsamen Mittelpunkt 24,25 rotierbar ist, ergeben sich folgende Freiheitsgrade für die Verstellung des Stabverbinders 2: Die Schenkel 10 sind entlang der Pfeile 40 axial bzgl. des Verbindungsteils 8 verschiebbar. Hierdurch können verschiedene Abstände zwischen den Wirbelsäulenstäben 4a,b ausgeglichen werden. Durch eine Rotation der Kugel 22 im Rahmen 20 um den Mittelpunkt 24,25 gemäß der Pfeile 42 sind die Längsachsen der Schenkel 10 zwischen paralleler und windschiefer Ausrichtung gegeneinander neigbar. Durch Rotation der Kugel 22 im Rahmen 20 um die Längsachse des Schenkels 10 der Klammer 6b in Richtung des Pfeils 44 ist außerdem eine Verdrehung der beiden Klammern 6a,b zueinander möglich. Hierdurch können alle während einer Operation an einem Patienten denkbaren Raumlagen bzw. Abweichungen der Parallelität der Wirbelsäulenstäbe zueinander ausgeglichen werden.

Das Verbindungsteil 8 kann im Rahmen des überlappenden Bereiches der Schenkel 10 frei in Richtung der Pfeile 40 verschoben werden. Verlaufen z.B. bei einer Operation bei eingesetztem Stabverbinder 2 die Schenkel 10 zwischen zwei eng aneinander liegenden Dornfortsätzen von Wirbeln, so kann das Verbindungsteil 8 aus der Mitte zur linken oder rechten Seite der Wirbelsäule hin verschoben werden, um die Dornfortsätze nicht zu berühren bzw. zu behindern.

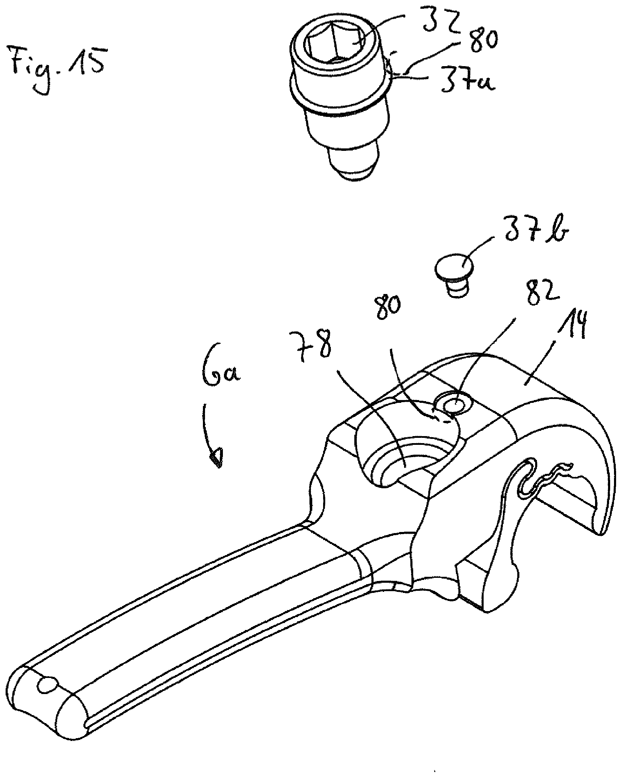

Bei Aufbringen der Klemme 14 auf den Wirbelsäulenstab 4a,b schwenkt deren Federbacken 18 um seine Lagerachse 28 in Richtung des Pfeils 30 vom Klemmbacken 16 weg, um den Wirbelsäulenstab 4a,b in der Klemme 14 aufzunehmen. Sobald der Wirbelsäulenstab 4a,b darin einliegt, schwenkt der Federbacken 18 entgegen der Richtung des Pfeils 30 wieder in die gezeigte Position zurück. Ein weiteres Bauteil der Klammer 6a,b, nämlich ein Fixierelement 32 in Form eines Gewindestiftes wird sodann in die Klemme 14 eingeschraubt, um gegen den Federbacken 18 zu drücken und diesen gegen den Wirbelsäulenstab 4a,b bzw. letzteren gegen den Klemmbacken 16 zu drücken und endgültig zu fixieren. Ähnlich zu oben ist auch das Fixierelement 32 mit einer Verliersicherung 33 ausgerüstet, um sich weder während der Handhabung noch nach Platzierung im Patienten vom restlichen Stabverbinder 2 lösen zu können (s. auch Fig.15). In Fig.2 ist die Verliersicherung der Übersichtlichkeit halber nicht dargestellt. Hierzu trägt das Fixierelement 32 einen radial vorstehenden Bund 37a und in den Grundträger 19 ist nach Einbringen des Fixierelements 32 ein Anschlagstift 37b eingebracht, z.B. verschweißt. Beim Herausdrehen des Fixierelements 32 stößt der Bund 37a an den Anschlagstift 37b und verhindert ein Lösen des Fixierelements 32 von der jeweiligen Klammer 6a,b.
Als Verliersicherung 33 ist alternativ auch - nicht dargestellt - eine in die Klemme 14 eingebrachte federnde Rastnase und ein Anschlag am Fixierelement 32 denkbar. Die Rastverbindung schnappt dann beim ersten Einschrauben des Fixierelements 32 ein, so dass dieses fortan nicht mehr entfernbar ist.
Die Klemmen 14 weisen zur Befestigung am Wirbelsäulenstab 4a,b damit eine Doppelfunktion auf. Die erste ist eine Federfunktion wie folgt: Durch einen teilweisen Freischnitt 46 eines Teils der einstückigen Klemme 14 entsteht der bewegliche Federbacken 18. Der restliche Teil der Klemme 14 bildet den feststehenden Klemmbacken 16. Dank des Federbackens 18 kann die Klemme 14 auf einen Wirbelsäulenstab 4a,b aufgeschnappt werden und auch von diesem wieder gelöst werden. Eine Federkraft F, mit der die Klemme 14 auf den Wirbelsäulenstab 4a,b wirkt, ist hierbei so gering, dass diese z.B. für eine grobe bzw. verschiebbare Halterung des Stabverbinders 2 auf den Wirbelsäulenstäben 4a,b ausreichend ist, so dass dieser zunächst nicht verrutschen oder vom Wirbelsäulenstab 4a,b gleiten kann. Die Federkraft F erlaubt jedoch, die Klemme 14 weiterhin auf dem Wirbelsäulenstab 4a,b händisch verdrehbar beziehungsweise verschiebbar oder auch wieder abnehmbar zu halten. Hierfür sind also keine Werkzeuge erforderlich.

Die Anlage der Klemmen 14 an den Wirbelsäulenstäben 4a,b erfolgt über jeweils drei radial einwärts ragende und parallel zu diesen verlaufende Wülste 15. Diese bewirken eine linienhafte Anlage der Klemme 14 an den Wirbelsäulenstäben 4a,b, um die bei der endgültigen Fixierung auftretenden Flächenpressungskräfte möglichst gering zu halten. Wirbelsäulenstäbe 4a,b aus Titan sind z.B. enorm druckempfindlich und können durch die gemäß Stand der Technik bekannte direkte Beaufschlagung mit einer Klemmschraube leicht beschädigt werden. Dies wird so vermieden.

Der Federbacken 18 ist an der Klemme über einen Federsteg 48 und ein Scharnier 49 verbunden. Beide Teile sind durch den Freischnitt 46 einstückig mit der Klemme 14 hergestellt. Das Scharnier 49 ist aus zwei sich gegenüberliegenden und durch einen Spalt 57 getrennten Formschlusselemente 55 gebildet und dient der Endfixierung, wenn nämlich dessen Scharnierteile durch Anziehen des Fixierelements 32 formschlüssig ineinander gedrückt werden. Der Federsteg 48 dient der Federung des Federbackens 18, damit dieser während des Aufschnappens auf den Wirbelsäulenstab 4a,b zurückfedert und in aufgeschnappter Position den Wirbelsäulenstab 4a,b zwischen sich und dem Klemmbacken 16 unter einer Federkraft F fixiert. Der Federsteg 48 bildet damit eine Federeinrichtung 47. Der Federsteg 48 besitzt eine Wellenform und ist damit auch in seiner Längsrichtung dehnbar. So wird beim Festziehen des Fixierelements 32 die Verschiebung des Federbackens 18, wenn das Scharnier 49 in den Formschluss gleitet, aufgefangen, d.h. eine Dehnung oder ein Abreißen des Federstegs 48 verhindert.
An ihren jeweiligen Enden 12b sind die Schenkel 10 mit einem Stift 34 versehen, welcher nach dem Zusammenbau des Stabverbinders 2 in die Schenkel 10 eingedrückt wird, auf deren Oberseite 35 verschweißt und anschließend verputzt wird. Auf Grund der Stifte 34 ist es nachträglich nicht mehr möglich, die Schenkel 10 aus dem Verbindungsteil 8 zu entfernen. So ist vermieden, dass während der Handhabung der Stabverbinder 2 in Einzelteile zerfällt.
Die Lage der Lagerachsen 28 befindet sich am Ende des Freischnittes 46. Die endgültige Fixierung auf dem Wirbelsäulenstab 4a,b stellt die zweite der Doppelfunktionen der Klemmen 14 dar. Ein händisches Verschieben oder Lösen ist dann nicht mehr möglich. Bei der Endfixierung, also dem Anziehen des Fixierelements 32 mit einem nicht dargestellten Werkzeug wird der Federbacken 18 in Richtung des Pfeils 51 gezwungen, wodurch das Scharnier 49 in Anschlag bzw. Formschluss gelangt. Mit anderen Worten wird im Bereich des Scharniers 49 der Federbacken 18 im Körper der Klemme 14 angepresst. Die Wellenstruktur des Federstegs 48 führt dazu, dass sich diese streckt und den erforderlichen Längenausgleich für das Verschieben des Federbackens 18 in Richtung des Pfeils 51 liefert. Die Klemme wird als Folge hiervon mit einer Haltekraft H gegen den Wirbelsäulenstab 4a,b gedrückt, die ein Lösen und Verschieben verhindert. Fixiereinrichtung 53 zur Endfixierung.

Der Schenkel 10 der Klammer 6b weist eine größere Länge l_{b} als der Schenkel 10 der Klammer 6a mit der Länge lₐ auf. So kann erreicht werden, dass bei Verschiebung der Schenkel 10 entlang der Pfeile 40 ein möglichst großer Abstandsbereich zwischen zu verbindenden Wirbelsäulenstäben 4a,b abgedeckt wird. Für den erreichbaren Minimalabstand liegen nämlich die Enden 12b einerseits in der Nähe des Fixierelements 32 der Klammer 6b und andererseits in der Nähe des Federbackens 18 der Klammer 6a. Da der jeweilige Federbacken 18 weiter als das Fixierelement 32 vom Verbindungsteil 8 entfernt ist, kann auch der Schenkel 10 der Klammer 6b mit einer größeren Länge l_{b} ausgeführt werden. Diese zusätzliche Längendifferenz l_{b}-lₐ liefert einen um dieses Stück erweiterten maximal möglichen Abstand für Wirbelsäulenstäbe 4a, b , wenn die Schenkel 10 bis zum Anschlag ihrer Stifte 34 am Verbbindungsteil 8 ausgezogen werden.

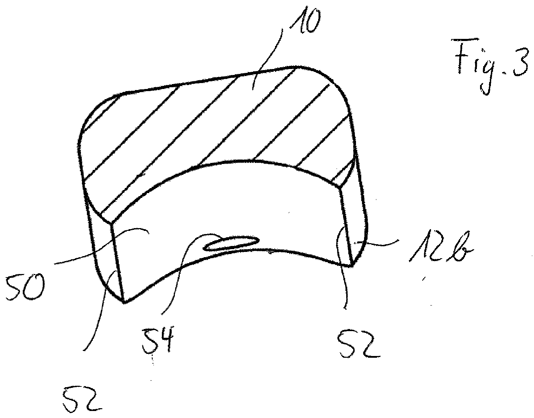

Fig.3 zeigt einen Schnitt entlang der Linie III-III durch den Schenkel 10 der Klammer 6a, also in Blickrichtung zu deren Ende 12b hin. Die der Kugel 22 bzw. deren Außenfläche 36 zugewandte Unterseite 50 des Schenkels 10 ist im Querschnitt kreisbogenförmig gestaltet, um sich an die kugelförmige Außenfläche 36 anzulegen. Der Kreisradius der Unterseite 50 ist hierbei passend zum Kugelradius der Kugel 22 gewählt, um eine flächenhafte Pressung zwischen Schenkel 10 und Kugel 22 zu erreichen.

Fig.3 zeigt die Klammer 6a im noch nicht montierten Zustand, d.h. bevor diese in das Verbindungsteil 8 eingesetzt wurde. Daher ist auch noch eine Öffnung 54 am Ende 12b zu erkennen, welche später zur Aufnahme des Stiftes 34 dient.

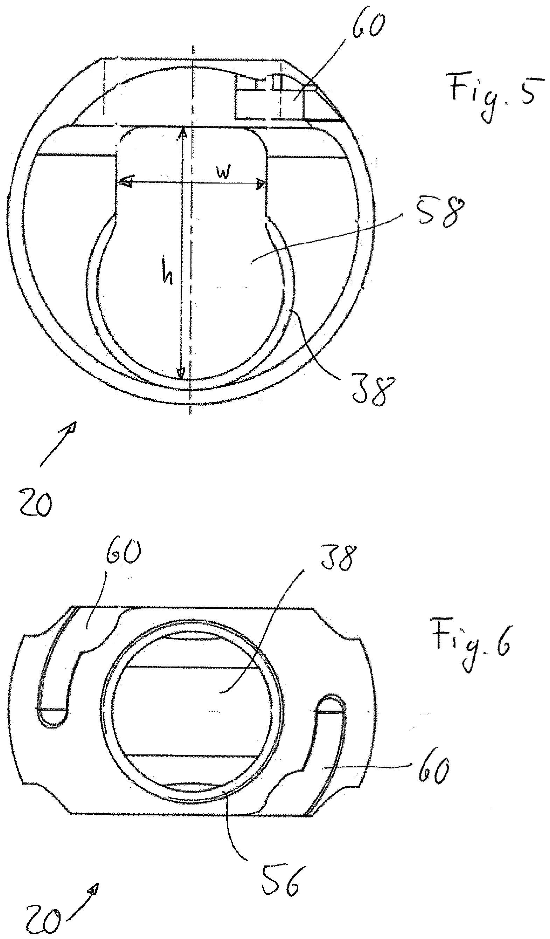

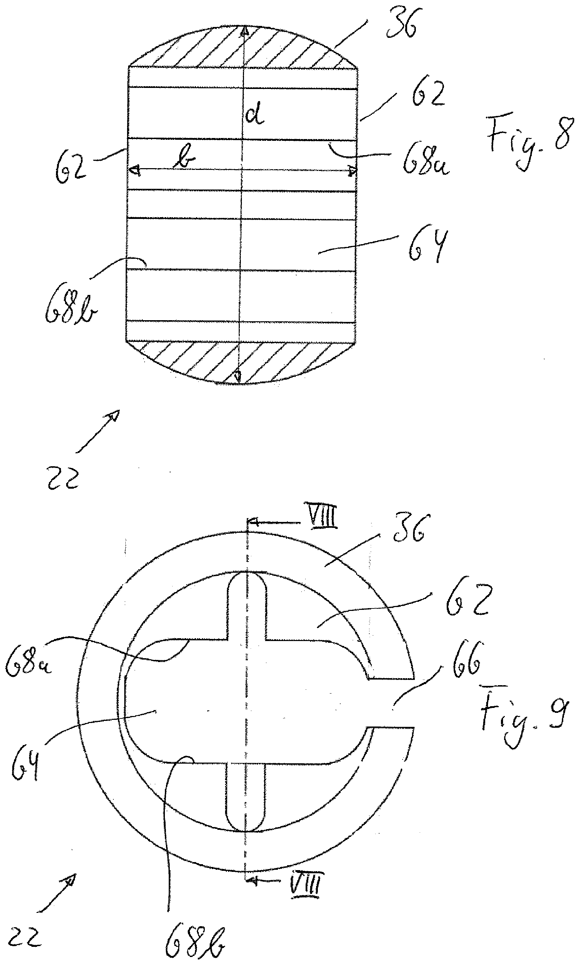

Die Figuren 4 - 6 zeigen den Rahmen 20. Zu erkennen ist insbesondere die Einschuböffnung 58, durch welche später die Kugel 22 ins Innere des Rahmens 20 bzw. zur Anlage an der Innenfläche 38 gebracht wird. Um die Kugel 22 einführen zu können, weist die Einschuböffnung 58 eine lichte Weite w sowie eine Höhe h auf, die jeweils größer entsprechender Dimensionen der Kugel 22 ist (siehe Fig. 8).
Ein Teil des Innenraums des Rahmens 20 bzw. der Einschuböffnung 58 ist die die nach Art eines Teils einer Kugeloberfläche ausgebildete Innenfläche 38. Der Rahmen 20 weist eine Gewindeöffnung 56 auf, in welche das Klemmelement 26 eingeschraubt wird.

An der Außenseite des Rahmens 20 ist die Gewindeöffnung 56 von einem Bajonettverschluss 60 umgeben. Dieser dient dazu, den Stabverbinder mit Hilfe eines nicht dargestellten Werkzeugs, z.B. eines speziellen Halters, das am Bajonettverschluss 60 angreift, zu greifen.
Die Figuren 7 - 9 zeigen die Kugel 22 mit ihrer teilkugelförmigen Außenfläche 36. Die Kugel 22 ist an zwei gegenüberliegenden Seiten nach Art einer Kugelschicht jeweils plan beschnitten, wodurch zwei parallele Planflächen 62 entstehen. Diese weisen einen senkrechten Abstand zueinander auf, d.h. die Kugel 22 besitzt eine Breite b. Die Breite b ist dabei kleiner der o.g. lichten Weite w der Einschuböffnung 58 bemessen, damit die Kugel 22 in entsprechender Richtung in die Einschuböffnung 58 geschoben werden kann.
Die größte Dimension der Kugel 22 ist ihr Durchmesser d, der so bemessen ist, dass er kleiner der Höhe h der Einschuböffnung 58 ist. Auch dies ist notwendig, damit die Kugel 22 in die Einschuböffnung 58 eingebracht werden kann.

Die Kugel 22 weist einen Aufnahmeraum 64 auf, in welchen gemäß der Figuren 1 und 2 der Schenkel 10 der Klammer 6b eingeführt wird. Um die oben erwähnte Klemmwirkung durch Druck auf die Außenfläche 36 bezüglich des einliegenden Schenkels 10 im Aufnahmeraum 64 zu erreichen, ist der Aufnahmeraum 64 seitlich durch einen Spalt 66 aufgebrochen. Die beiden Kugelhälften, welche sich bezüglich einer durch den Spalt 66 verlaufenden Symmetrieebene der Kugel 22 gegenüberliegen, können so aufeinander zu federn, wodurch bei äußerem Druck auf die Kugel 22 zwischen den Innenflächen 68a,b eine den Schenkel 10 fixierende Klemmwirkung entsteht.

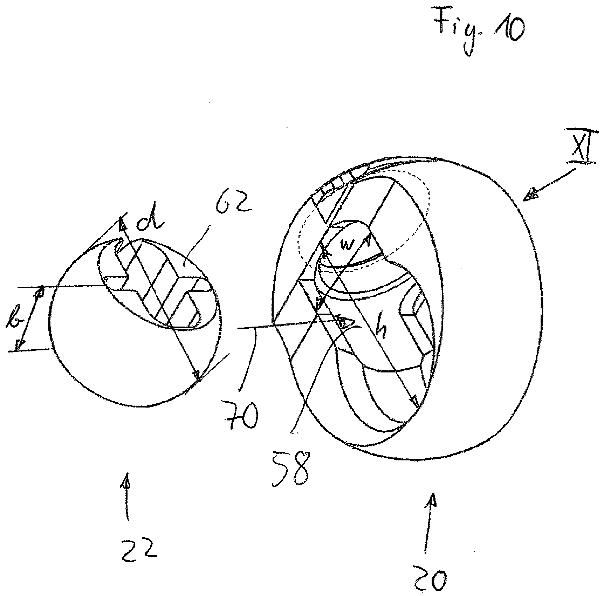

Die Figuren 10 bis 14 zeigen die Montage der Kugel 22 in den Rahmen 20 in chronologischer Abfolge. Fig. 10 zeigt die Ausgangsposition für die Montage, bei welcher die Kugel 22 so ausgerichtet ist, dass deren Planflächen 62 parallel zur Einschubrichtung (Richtung des Pfeils 70) ausgerichtet sind. Anders ausgedrückt, liegt die Kugel 22 so, dass die Planflächen 62 mit der Erstreckungsrichtung der Einschuböffnung 58 im Rahmen 20 fluchten. Da die Breite b kleiner der Weite w und der Durchmesser d kleiner der Höhe h gewählt ist, gelingt es, die Kugel 22 durch die Einschuböffnung 58 in Richtung des Pfeils 70 ins Innere des Rahmens 20 zu verbringen.

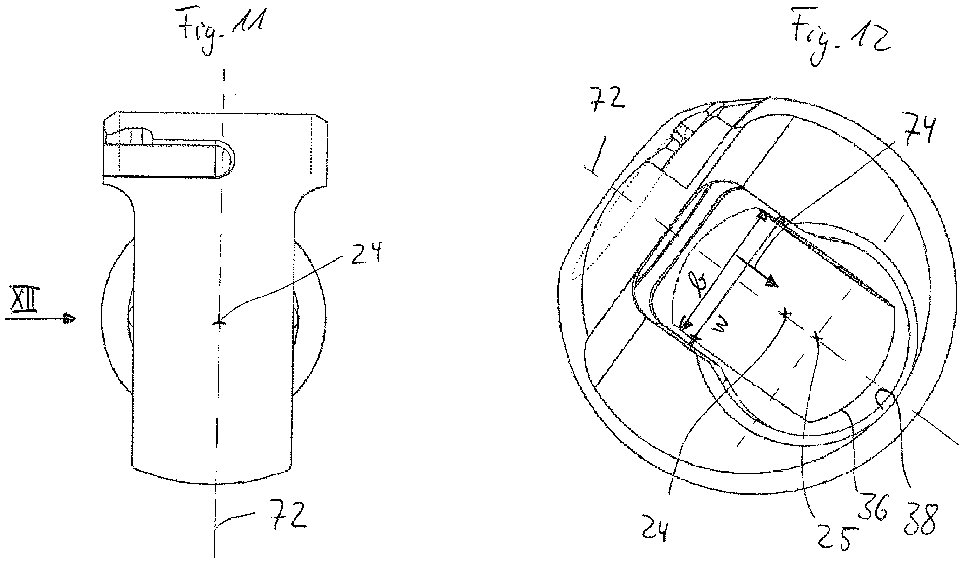

Die Figuren 11 und 12 zeigen die Situation nach Ende des Einschubvorganges, wenn also der Mittelpunkt 24 der Kugel 22 sich in der Mitte (angedeutet durch die Mittenachse 72) des Rahmens 20 befindet.

In Fig. 12 ist hierbei zu erkennen, dass der Einschub der Kugel 22 bezüglich deren Mittelpunkt 24 etwas oberhalb des Kugelmittelpunktes 25 der Innenfläche 38 erfolgt. Dies ist nicht anders möglich, da sonst die Außenfläche 36 der Kugel 22 mit der Innenfläche 38 kollidieren würde. Der Mittelunkt 24 der Kugel 22 befindet sich daher nach erfolgtem Einschieben in das Verbindungsteil 8 auf der Mittenachse 72 noch oberhalb des Mittelpunktes 25 der kugelförmigen Innenfläche 38.

Fig. 13 zeigt daher den nächsten Montageschritt: Die Kugel 22 wird in Richtung des Pfeils 74 entlang der Mittenachse 72 abgesenkt, bis die Außenfläche 36 an der Innenfläche 38 an der Unterseite 75 des Verbindungsteils 8 anliegt. Das Absenken ist möglich, da wegen der immer noch vorhandenen Drehstellung der Kugel 22 nur ein kleiner Bereich 73 der Außenfläche 36 mit der Innenfläche 38 in Berührung kommt und die restliche Kugel 22 genügend Abstand bzw. Bewegungsspielraum zur Innenfläche 38 besitzt. Die Mittelpunkte 24 und 25 von Kugel 22 und Innenfläche 38 fallen nun zusammen. Daher kann nun die Kugel 22 in jeder beliebigen Richtung um den Mittelpunkt 24,25 rotiert werden. Hierbei gleiten große Teile der Außenfläche 36 auf die Innenfläche 38 auf. Außerdem ergibt sich so oberhalb der Kugel 22 der Aufnahmeraum 76 zur Aufnahme des Schenkels 10, wie in den Figuren 1 und 2 gezeigt. Der Schenkel 10 ist im Aufnahmeraum 76 ebenfalls nach Art einer Gleitführung alleine in Richtung des Pfeils 40 axial verschiebbar geführt. Durch nahezu spielfreies Einlegen eines Schenkels 10 in den Aufnahmeraum 76 wird außerdem verhindert, dass sich die Kugel 22 entgegen der Richtung des Pfeils 74 verschiebt. So ist eine Überwindung der durch die Innenfläche 38 erzwungenen Formschlusses nicht mehr möglich und die Kugel 22 ist im Verbindungsteil 8 fixiert.

Fig. 14 zeigt, wie insbesondere die Kugel 22 um etwa 90° um die Mittenachse 72 rotiert wird, so dass die Planflächen 62 nun etwa parallel zur Querschnittsfläche der Einschuböffnung 58 liegen. So ist auch der Aufnahmeraum 64 zugänglich zur Aufnahme eines ersten Schenkels 10. Die möglichen Bewegungen der Kugel 22 sind wieder angedeutet durch die Pfeile 42 bzw. den Pfeil 44. Fig. 14 zeigt nun eine Relativlage von Kugel 22 und Rahmen 20, die gegenüber der aus Fig. 1 und 2 noch um 90° in Richtung des Pfeils 44 verdreht ist.

Durch die Verdrehung der Kugel 22 zwischen den in Fig. 13 und 14 gezeigten Positionen ist in Fig. 14 die Kugel 22 in der Innenfläche 38 fixiert, so dass diese nicht mehr in Richtung des Pfeils 70 verschiebbar ist. Hierzu müsste sie außerdem zunächst in die Position gemäß Fig. 13 zurückgebracht werden, d.h. die Planflächen 62 wieder entsprechend ausgerichtet werden.

Fig. 15 zeigt nochmals die Verliersicherung 33 im Detail im noch nicht montierten Zustand. Gezeigt ist nochmals beispielhaft die Klammer 6a aus Fig.1, die eine Öffnung 78 zur Aufnahme des Fixierelements 32 und eine Öffnung 82 zur Aufnahme des Anschlagstiftes 37b aufweist. Zu sehen ist insbesondere der Bund 37a am Fixierelement 32 und der mit diesem zusammenwirkende Anschlagstift 37b. Zuerst wird das Fixierelement in die Öffnung 80 eingeschraubt, bis der Bund 37a in der Öffnung 78 versenkt ist. Dann wird der Anschlagstift 37b in der Öffnung 82 fixiert, z.B. durch Einschweißen. Die gestrichelte Linie 80 deutet an, dass dann der Anschlagstift 37b den Bund 37a überdeckt. Beim Ausschrauben des Fixierelements 32 stößt der Bund 37a dann an den Anschlagstift 37b, bevor das Fixierelement 32 die Öffnung 78 verlassen kann und ist so dauerhaft dort gehalten.

## Patentansprüche

1. Stabverbinder (2) zur Querverbindung zweier Wirbelsäulenstäbe (4a,b),
- mit zwei, in einem Montagezustand je einen Wirbelsäulenstab (4a,b) befestigend teilumgreifenden Klemmen (14),
- mit einem die Klemmen (14) relativ zueinander ortsfixierenden Quersteg (11), der zwei, an je einer der Klemmen (14) angebrachte Schenkel (10), und ein von beiden Schenkeln (10) durchsetztes Verbindungsteil (8) mit einer auf beide Schenkel (10) einwirkenden und einen arretierenden Reibschluss zwischen den Schenkeln (10) und dem Verbindungsteil (8) erzeugenden Arretiervorrichtung (31) enthält,
- mit einer Federeinrichtung (47) für die Klemme (14), deren Federkraft (F) derart gering ist, dass die Klemme (14) händisch auf den Wirbelsäulenstab (4a,b) aufschnappbar, im Montagezustand auf diesem verschiebbar und von diesem lösbar ist,
- mit einer von der Federeinrichtung (47) unabhängig betätigbaren Fixiereinrichtung (53) für die Klemme (14), deren Haltekraft (H) derart groß ist, dass die Klemme (14) im Montagezustand auf dem Wirbelsäulenstab (4a,b) fest fixiert ist,
**dadurch gekennzeichnet, dass**
das Verbindungsteil (8) einen Rahmen (20) zur Aufnahme des ersten Schenkels (10) und eine im Rahmen rotierbar gelagerte, vom zweiten Schenkel (10) durchsetzte Kugel (22) enthält.

2. Stabverbinder (2) nach einem der vorhergehenden Ansprüche, bei dem die Kugel (22) einen zur Arretierung des zweiten Schenkels (10) komprimierbaren Spalt (66) aufweist.

3. Stabverbinder (2) nach Anspruch 3, bei dem die Arretiervorrichtung (31) ein sich am Rahmen (20) abstützendes und den ersten Schenkel (10) gegen die Kugel (22) pressendes Klemmelement (26) enthält.

4. Stabverbinder (2) nach einem der vorhergehenden Ansprüche, bei dem die Klemme (14) einen Grundträger (19) mit einem feststehenden Klemmbacken (16) und einen am Grundträger (19) federnd gelagerter Federbacken (18) enthält.

5. Stabverbinder (2) nach Anspruch 4, bei dem die Federeinrichtung (47) ein den Federbacken (18) mit dem Grundträger (19) verbindender Federsteg (48) ist.

6. Stabverbinder (2) nach Anspruch 4 oder 5, bei dem Federbacken (18), Federsteg (48) und Grundträger (19) einstückig ausgebildet sind.

7. Stabverbinder (2) nach Anspruch 6, bei dem der Federbacken (18) und/oder der Federsteg (48) aus dem Grundträger (19) freigeschnitten ist.

8. Stabverbinder (2) nach einem der Ansprüche 4 bis 7, bei dem der Federbacken (18) und der Grundträger (19) je ein Formschlusselement (55) aufweisen, die bezüglich der Federeinrichtung (47) Spiel zueinander aufweisen und durch die Fixiereinrichtung (53) formschlüssig ineinander bewegbar sind.

9. Stabverbinder (2) nach Anspruch 8, bei dem die Formschlusselemente (55) an ihren einander zugewandten Seiten eine zumindest annähernd S-förmige Passform aufweisen.

10. Stabverbinder (2) nach einem der vorhergehenden Ansprüche, bei dem die Fixiereinrichtung (53) ein das Aufschwenken der Klemme (14) verhinderndes Fixierelement (32) enthält.

11. Stabverbinder (2) nach Anspruch 10, bei dem das Fixierelement (32) ein auf die Klemme (14) einwirkendes Gewindeelement enthält.

## Claims

1. Rod connector (2) for transversely connecting two spinal rods (4a, b),
- having two clamps (14) that, in a mounted state, each partially fixedly enclose a spinal rod (4a, b),
- having a crossbar (11) that fixes the clamps (14) in place relative to one another and includes two branches (10) each mounted on one of the clamps (14), and a connection part (8) that both the branches (10) pass through, said connection part (8) having a locking device (31) that acts on both branches (10) and produces a locking frictional connection between the branches (10) and the connection part (8),
- having a spring device (47) for the clamp (14), the spring force (F) thereof being low enough that the clamp (14) is able to be manually snapped onto the spinal rod (4a, b), is able to adjusted on this in the mounted state and is able to be detached from this,
- having a fixing device (53) for the clamp (14) that is able to be activated independently of the spring device (47), the holding force (H) of said fixing device (53) being so large that the clamp (14) is solidly fixed on the spinal rod (4a, b) in the mounted state,
**characterised in that**
the connection part (8) includes a frame (20) for receiving the first branch (10) and a ball (22) which is rotatably mounted in the frame and passed through by the second branch (10).

2. Rod connector (2) according to one of the preceding claims, wherein the ball (22) has a compressible gap (66) for locking the second branch (10).

3. Rod connector (2) according to claim 3, wherein the locking device (31) includes a clamping element (26) that is supported on the frame (20) and presses the first branch (10) against the ball (22).

4. Rod connector (2) according to one of the preceding claims, wherein the clamp (14) includes a base support (19) having a stationary clamping jaw (16) and a spring jaw (18) which is spring-mounted on the base support (19).

5. Rod connector (2) according to claim 4, wherein the spring device (47) is a spring rod (48) that connects the spring jaw (18) to the base support (19).

6. Rod connector (2) according to claim 4 or 5, wherein the spring jaw (18), the spring rod (48) and the base support (19) are formed as one piece.

7. Rod connector (2) according to claim 6, wherein the spring jaw (18) and/or the spring rod (48) is cut out of the base support (19).

8. Rod connector (2) according to one of claims 4 to 7, wherein the spring jaw (18) and the base support (19) each have a positive-locking element (55) that, with regards to the spring device (47), have clearance relative to one another and are able to be moved into one another by the fixing device (53) to have positive locking.

9. Rod connector (2) according to claim 8, wherein the positive-locking elements (55) have at least one approximately S-shaped matching form on their sides facing towards one another.

10. Rod connector (2) according to one of the preceding claims, wherein the fixing device (53) includes a fixing element (32) that prevents the clamp (14) from swinging open.

11. Rod connector (2) according to claim 10, wherein the fixing element (32) includes a threaded element that acts on the clamp (14).

## Revendications

1. Connecteur (2) de tiges de liaison transversale de deux tiges (4a, b) de colonne vertébrale,
- comprenant deux pinces (14), prenant partiellement dans un état de montage respectivement une barre (4a, b) de colonne vertébrale en la fixant,
- comprenant une traverse (11), qui immobilise en place les pinces (14), l'une par rapport à l'autre, et qui comporte deux branches (10) mises sur respectivement l'une des pinces (14) et une partie (8) de liaison traversée par les deux branches (10) et ayant un dispositif (31) de blocage, agissant sur les deux branches (10) et produisant un frottement de blocage entre les branches (10) et la partie (8) de liaison,
- comprenant un dispositif (47) à ressort pour la pince (14), dont la force (F) de ressort est petite, de manière à ce que la pince (14) puisse être mise à la main sur la tige (4a, b) de colonne vertébrale, être déplacée sur celle-ci à l'état monté et en être détachée,
- comprenant un dispositif (53) d'immobilisation de la pince (14), qui peut être actionné indépendamment du dispositif (47) à ressort et dont la force (H) de maintien est si grande que la pince (14), à l'état monté, est immobilisée fixement sur la tige (4a, b) de colonne vertébrale,
**caractérisé en ce que**
la partie (8) de liaison comporte un cadre (20) de réception de la première branche (10) et une bille (22), montée tournante dans le cadre et traversée par la deuxième branche (10).

2. Connecteur (2) de tiges suivant l'une des revendications précédente, dans lequel la bille (22) a une fente (66) pouvant être comprimée pour bloquer la deuxième branche (10).

3. Connecteur (2) de tiges suivant la revendication 2, dans lequel le dispositif (31) de blocage comporte un élément (26) de serrage s'appuyant sur le cadre (20) et pressant la première branche (10) sur la bille (22).

4. Connecteur (2) de tiges suivant l'une des revendications précédentes, dans lequel la pince (14) comporte un support (19) de base, comprenant une mâchoire (16) de serrage fixe et une mâchoire (18) à ressort, montée élastiquement sur le corps (19) de base.

5. Connecteur (2) de tiges suivant la revendication 4, dans lequel le dispositif (47) à ressort est une âme (48) à ressort reliant la mâchoire (18) élastique au support (19) de base.

6. Connecteur (2) de tiges suivant la revendication 4 ou 5, dans lequel la mâchoire (18) élastique, l'âme (48) à ressort et le support (19) de base, sont d'une seule pièce.

7. Connecteur (2) de tiges suivant la revendication 6, dans lequel la mâchoire (18) élastique et/ou l'âme (48) à ressort sont découpées dans le support (19) de base.

8. Connecteur (2) de tiges suivant l'une des revendications 4 à 7, dans lequel la mâchoire (18) élastique et le support (19) de base ont respectivement un élément (55) à complémentarité de forme, qui, par rapport au dispositif (47) à ressort, ont un jeu l'un par rapport à l'autre et peuvent être déplacés l'un dans l'autre à complémentarité de forme par le dispositif (53) d'immobilisation.

9. Connecteur (2) de tiges suivant la revendication 8, dans lequel les éléments (55) à complémentarité de forme ont, sur leurs côtés tournés l'un vers l'autre, une contre-forme au moins à peu près en forme de S.

10. Connecteur (2) de tiges suivant l'une des revendications précédentes, dans lequel le dispositif (53) d'immobilisation comporte un élément (32) d'immobilisation empêchant la pince (14) de pivoter.

11. Connecteur (2) de tiges suivant la revendication 10, dans lequel l'élément (32) de fixation comporte un élément fileté agissant sur la pince (14).
